# EUROPEAN PATENT APPLICATION

(11) **EP 2 286 788 A2**
(43) Date of publication of application: **23.02.2011**
(21) Application number: 10172938.2
(22) Date of filing: 16.08.2010
(51) Int. Cl.: A61K 8/34, A61K 8/36, A61K 8/86, A61Q 9/00, B26B 21/44

(54) **Hair removal device**

(30) Priority: 21.08.2009 EP 09168358
(71) Applicant: The Gillette Company, Boston, MA 02127 (US)
(72) Inventor: Stephens, Alison, Fiona, Maidenhead, Berkshire SL6 9LA (GB); Geary, Elaine, Alice, Marie, St Margarets, Middlesex TW1 2QX (GB); Kwiecien, Michael, Joseph, Scituate, MA 02066 (US)
(74) Representative: Wilding, Richard Alan

(57) **Abstract**

According to the invention, a hair removal device is provided comprising a solid lubricating composition, the solid lubricating composition comprising:
a. from 40% to 95% by weight of the solid lubricating composition of a water-soluble lubricating polymer which:
i. is solid at STP;
ii. has a molecular weight of less than or equal to 8,000,000, preferably from 100,000 to 3,000,000;

b. from 3 to 50%, preferably from 5 to 25%, by weight of the solid lubricating composition of one or more fatty alcohols, selected from C₁₂ - C₃₀ fatty alcohols.

## Description

### FIELD OF THE INVENTION

The present invention concerns the provision of a hair removal device comprising robust solid lubricating compositions.

### BACKGROUND OF THE INVENTION

Hair removal devices comprising a soap composition are known - reference is made to WO 07/056509. WO 07/056509 also variously teaches to include a small amount of polymer, such as polyoxyethylene, and fatty alcohol, such as behenyl alcohol, within the soap composition. It is also known to provide a hair removal device incorporating a skin-engaging composition comprising large quantities of hydrophilic polymers, such as polyethylene oxide, to lubricate the skin. Reference is made, by way of example, to WO 97/02116 and WO 97/02117. A disadvantage of the compositions of '116 and '117 is that they may be prone to disintegrate in water and therefore be unsuited to provide lubrication for more than a few shaves.

### SUMMARY OF THE INVENTION

According to the invention, a hair removal device is provided comprising a solid lubricating composition, the solid lubricating composition comprising:
a. from 40% to 95% by weight of the solid lubricating composition of a water-soluble lubricating polymer which:
   i. is solid at STP;
   ii. has a molecular weight of less than or equal to 8,000,000, preferably from 100,000 to 3,000,000;
b. from 3 to 50%, preferably from 5 to 25%, by weight of the solid lubricating composition of one or more fatty alcohols, selected from C₁₂ - C₃₀ fatty alcohols.

### BRIEF DESCRIPTION OF THE DRAWINGS

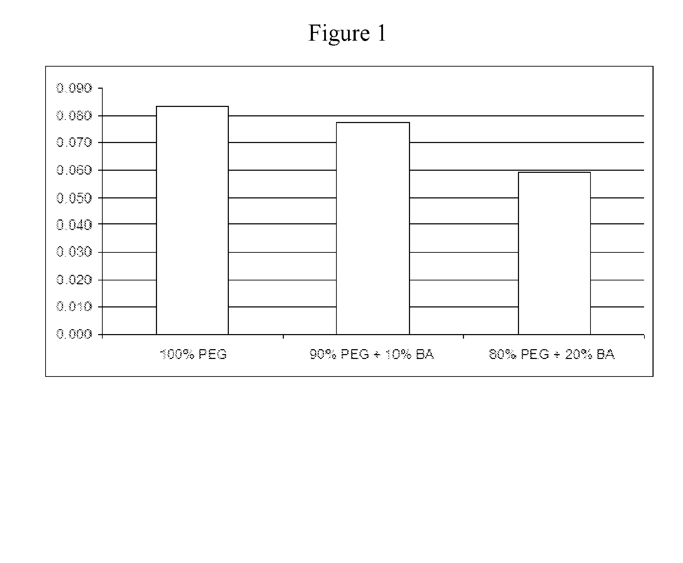

Figure 1 is a graph of PEG released into a solvent as discussed in the Disintegration/dissolution Test, below.

### DETAILED DESCRIPTION OF THE INVENTION

The present invention overcomes problems associated with prior art hair removal devices incorporating comprise a solid lubricating composition comprising hydrophilic lubricating polymer. The inclusion of the defined fatty alcohols renders the compositions more resistant to dissolution in water and therefore enhances their longevity - in other words, it enables them to provide lubrication for a greater number of uses than would otherwise be the case.

As used herein, the term "STP", or "Standard Temperature and Pressure", refers to a temperature of 20 °C and a pressure of 101.325 kPa.

The hair removal device according to the invention may be any hair removal device, such as, but not limited to, a razor or a hair removal device comprising a light source to degrade or destroy the hair. If the hair removal device comprises a light source, then the light source is advantageously a source of laser light. Preferably, the hair removal device is a razor. In the case of a razor, then the solid lubricating composition may advantageously be provided on the razor cartridge, and is preferably as a strip located before and/or after the blade(s) in the direction of cutting. WO 97/02116, referred to above, illustrates locations in which such a strip may be placed.

According to the invention, the solid lubricating composition may comprise from 3 to 50%, preferable from 5 to 25% by weight of the solid lubricating composition of one or more fatty alcohols selected from C₁₂ - C₃₀, preferably C₁₂ - C₂₂ fatty alcohol. More preferably, the fatty alcohol comprises cetyl, stearyl, or behenyl alcohol or mixtures thereof. The presence of the defined amount of fatty alcohol may enhance the life of the composition by reducing its tendency to disintegrate by dissolution in water, as illustrated in Figure 1, discussed below. If too much fatty alcohol is present, however, then the solid lubricating composition may become too hydrophobic and the water soluble lubricating polymer may be prevented from performing its intended function. In addition, as discussed below, if the solid lubricating composition is cold-pressed, then it advantageously does not comprise more than 20% and preferably less than 15% of fatty alcohol by weight of the solid lubricating composition. This is because, as the amount of fatty alcohol increases, the solid lubricating composition becomes increasingly brittle to the extent that it cannot properly be affixed to a hair removal device or may break in use.

The solid lubricating composition, comprises a water-soluble lubricating polymer. The water-soluble lubricating polymer may achieve lubrication by a number of mechanisms, such as binding water to form a gel. In order to be effective, the polymer must be solid at STP and have a molecular weight of less than 8,000,000. Preferably, it has a molecular weight from 100,000 to 7,000,000. Suitable water-soluble lubricating polymers include polyalkylene glycol; cellulosic polymers, including hydroxypropyl cellulose; hydroxypropyl methylcellulose (HPMC); polyvinyl pyrrolidone; polyacrylamide; polyvinyl imidazoline; polyhydroxyethylmethacrylate and mixtures thereof. As used herein, "polyalkylene glycol" is synonymous with "polyalkane oxide", although some sources have, in the past, referred to lower molecular weight variants as "polyalkylene glycol" and higher molecular weight variants as "polyalkane oxide". Preferably, the polyalkylene glycol (polyalkane oxide) comprises polyethylene glycol (which may also be referred to herein as "PEG"), polypropylene glycol, or mixtures thereof and more preferably it comprises polyethylene glycol.

The solid lubricating composition preferably comprises from 40 to 95%, preferably from 45 to 85%, by weight of water-soluble lubricating polymer.

Advantageously, the water-soluble lubricating polymers comprise polyethylene oxides generally known as POLYOX (available from the Dow Chemical Company) or
ALKOX (available from Meisei Chemical Works, Kyoto, Japan). In one embodiment, these particular polyethylene oxides will have molecular weights of about 100,000 to about 6 million, preferably from about 300,000 to about 5 million and the polyethylene oxide comprises a blend of polyethylene oxide having an average molecular weight of about 5 million and polyethylene oxide having an average molecular weight of about 300,000.

Advantageously, the solid lubricating composition comprises less than 2%, preferably less than 1% and more preferably 0% of water-soluble surfactant, by weight of the solid lubricating composition. As used herein, the term "water-soluble surfactant" refers to surfactant which fulfils the following condition: at 25°C, 1g of the surfactant dissolves in 1000ml or less of water. For completeness, one requires more than 10,000ml of water at 25°C in order to dissolve 1g of magnesium stearate or 1g of cetyl alcohol.

Advantageously, the solid lubricating compositions may comprise a hydrophobic binder. The presence of such a component may enhance the life of the composition by reducing its tendency to be mechanically eroded. Advantageously, the hydrophobic binder is solid at STP. Suitable hydrophobic binders include divalent metal cation stearate, preferably magnesium stearate, calcium stearate, zinc stearate, or mixtures thereof, more preferably magnesium stearate; ethyl cellulose; polycaprolactone; polyethylene; polypropylene; polystyrene; butadiene-styrene copolymer (e.g. medium and high impact polystyrene); polyacetal; acrylonitrilebutadiene-styrene copolymer; ethylene vinyl acetate copolymer and blends such as polypropylene/polystyrene blend; and mixtures thereof. In the event that the solid lubricating composition is cold-pressed, then it preferably comprises from 1 to 20% and more preferably from 5 to 15% hydrophobic binder, by weight of the solid lubricating composition. In the event that the solid lubricating composition is manufactured by an extrusion process, as discussed below, then it preferably comprises from 10 to 50%, more preferably from 15 to 40% and yet more preferably 20 to 35% hydrophobic binder, by weight of the solid lubricating composition.

It may be difficult to provide a perfumed a solid lubricating composition of the present type, because perfumes are liquids and are therefore difficult to retain within distribute evenly throughout the solid matrix during the manufacturing process. For example, in a high temperature extrusion process, perfume oils may volatilize and boil off. During a cold-pressing process, the perfume may be squeezed out during compaction and lost as well. Furthermore, perfume oils are usually fairly hydrophobic and may be repelled by hydrophilic materials, such as the water-soluble lubricating polymers disclosed herein. The result tends to be an unsatisfactorily perfumed product.

In order to overcome this disadvantage, perfume oils comprised within the solid lubricating compositions according to the invention may be releasably encapsulated within a powder. Any powder which permits perfume release in use, such as on contact with water, may suitably be used. Advantageously, the powder comprises a cyclic oligosaccharide, which may encapsulate the perfume.

As used herein, the term "cyclic oligosaccharide" means a cyclic structure comprising six or more saccharide units. Preferred for use herein are cyclic oligosaccharides having six, seven or eight saccharide units and mixtures thereof, more preferably six or seven saccharide units and even more preferably seven saccharide units. It is common in the art to abbreviate six, seven and eight membered cyclic oligosaccharides to α, β and γ respectively.

Cyclic oligosaccharides for use herein may comprise any suitable saccharide or mixtures of saccharides. Examples of suitable saccharides include, but are not limited to, glucose, fructose, mannose, galactose, maltose and mixtures thereof, preferably glucose. The preferred cyclic oligosaccharides for use herein are α-cyclodextrins or β- cyclodextrins, or mixtures thereof, and the most preferred cyclic oligosaccharides for use herein are β-cyclodextrins.

The cyclic oligosaccharides for use herein may be substituted by any suitable substituent or mixture of substituents. Herein the use of the term "mixture of substituents" means that two or more different suitable substituents can be substituted onto one cyclic oligosaccharide. The derivatives of cyclodextrins consist mainly of molecules wherein some of the OH groups have been substituted. Suitable substituents include, but are not limited to, alkyl groups; hydroxyalkyl groups; dihydroxyalkyl groups; (hydroxyalkyl) alkylenyl bridging groups such as cyclodextrin glycerol ethers; aryl groups; maltosyl groups; allyl groups; benzyl groups; alkanoyl groups; cationic cyclodextrins such as those containing 2-hydroxy-3- (dimethylamino) propyl ether; quaternary ammonium groups; anionic cyclodextrins such as carboxyalkyl groups, sulphobutylether groups, sulphate groups, and succinylates; amphoteric cyclodextrins; and mixtures thereof.

The substituents may be saturated or unsaturated, straight or branched chain. Preferred substituents include saturated and straight chain alkyl groups, hydroxyalkyl groups and mixtures thereof. Preferred alkyl and hydroxyalkyl substituents are selected from C1-C8 alkyl or hydroxyalkyl groups or mixtures thereof, more preferred alkyl and hydroxyalkyl substituents are selected from C1-C6 alkyl or hydroxyalkyl groups or mixtures thereof, even more preferred alkyl and hydroxyalkyl substituents are selected from C1-C4 alkyl or hydroxyalkyl groups and mixtures thereof. Especially preferred alkyl and hydroxyalkyl substituents are propyl, ethyl and methyl, more especially hydroxypropyl and methyl and even more preferably methyl.

Preferred cyclic oligosaccharides for use in the present invention are unsubstituted, or are substituted by only saturated straight chain alkyl, or hydroxyalkyl substituents. Therefore, preferred examples of cyclic oligosaccharides for use herein are α-cyclodextrin, β-cyclodextrin, methyl-α-cyclodextrin, methyl-α-cyclodextrin, and hydroxypropyl-β-cyclodextrin.

The solid lubricating composition comprised within the hair removal device according to the invention may be manufactured in a number of ways. Traditionally, such a composition may be manufactured using an extrusion method, such as is disclosed in WO 97/02116 and WO 97/02117, referred to above. If the solid lubricating composition comprises a temperature-sensitive component, such as a fragrance oil, then the solid lubricating composition may advantageously be a cold-pressed composition, as exemplified in Examples 1 to 3 below. By cold-pressing, temperature-sensitive components may be better retained within the composition and not lost by boiling off, degradation, and the like. As discussed above, if the composition is cold-pressed, then it advantageously does not comprise more than 20% and preferably less than 15% of fatty alcohol by weight of the solid lubricating composition.

As used herein, the term "cold-pressed" means that the formulation has been manufactured by process involving a compression step, without any active heating and the term "cold-pressing" should be interpreted accordingly. For the avoidance of doubt, heat generated by the compression step itself is not regarded as being active heating.

The term "temperature-sensitive component" refers to materials which have a boiling point, undergo significant sublimation, or which undergo a change in chemical structure at temperatures above ambient temperatures and below 100°C. Materials having a boiling point in this range are known to the skilled person and include many low boiling point perfume oils. Low boiling perfume oils, or top notes, are those that provide the initial burst of fragrance following application to the skin, because they boil off quickly when exposed to body heat. The skilled person is similarly familiar with materials which undergo significant sublimation in the present temperature range. An example of a commonly used sensate which sublimes in this temperatures range is menthol. Materials whose chemical structures change at temperatures within the cited range are known to the skilled person. One example of such a material is L-ascorbic acid, which oxidizes at an increasing rate as the temperature rises.

The following, non-limiting examples, illustrate the manufacture of solid lubricating compositions:

### Example 1

| **Component** | **Wt%** |
|---|---|
| | |
| PEG¹ | 90 |
| Behenyl alcohol² | 10 |

### Example 2

| **Component** | **Wt%** |
|---|---|
| | |
| PEG¹ | 80 |
| Behenyl alcohol² | 20 |

### Example 3

| **Component** | **Wt%** |
|---|---|
| PEG¹ | 80 |
| Behenyl alcohol² | 10 |
| Magnesium Stearate | 10 |

| | |
|---|---|
| ¹Polyox^{™} WSR-N750 powder, a polyethylene glycol with molecular weigh about 300,000 manufactured by Dow Chemicals ²Stenol-1822 powder manufactured by Cognis | |

The compositions of Examples 1 -3 are manufactured by placing the materials into a blending device and blending until the mixture is entirely homogeneous. Once homogenised, the mixture is pressed into the desired shape using an appropriate mould tool / punch. An example of a suitable device is the GEA Courtoy R253-27 tablet press. The powder is compressed using a force of 1 x 10¹⁰ - 1 x 10¹² Nm⁻². The resultant tablet is ejected from the mould in the desired shape.

### Example 4

| **Component** | **Wt%** |
|---|---|
| Cetyl Alcohol | 10.0 |
| Stearyl Alcohol | 10.0 |
| PEG-115M¹ | 28.53 |
| PEG-7M² | 18.18 |
| Styrene/Butadiene Copolymer³ | 23.55 |
| PEG-100⁴ | 4.50 |
| BHT | 0.24 |
| Polycaprolactone | 5.00 |

| | |
|---|---|
| ¹ Polyox Coag., manufactured by Dow Chemical ² Polyox N750, manufactured by Dow Chemical ³ 731 HIPS (High Impact Polystyrene) manufactured by BASF ⁴ Carbowax PEG | |

The above composition is manufactured by extruding the blend through a Haake System 90, 0.019m (¾ inch) diameter extruder with a barrel pressure of about 68.9 - 137.8 bar (1000-2000 psi), a rotor speed of about 10 to 50 rpm, and a temperature of about 150-185"C and a die temperature of about 170-185"C. The extruded strip of composite is cooled and sliced to the appropriate size.

### Disintegration/dissolution Test

The following identically sized and shaped strip samples were manufactured:
- A sample of 100% PEG (Polyox^{™} WSR-N750);
- A solid lubricating composition to Example 1
- A solid lubricating composition to Example 2

In each case, a 10µl drop of extraction solvent (Deuterium oxide) was placed at the bottom of a glass vial and the strip of solid lubricant placed on the drop of solvent. The vial was then placed into an oven at 50°C for two minutes which 'glued' the fragment to the vial. 1000µl of extraction solvent (Deuterium oxide) was added to the vial and it was tumble mixed for 20 minutes. After 20 minutes, 200ul of the solution was extracted from the top of the vial for NMR analysis of PEG levels. The results are presented in Figure 1, in which illustrates PEG released into the solvent:
- the term "Polyox" refers to the Polyox^{™} WSR-N750, powdered polyethylene glycol;
- the term "BA" refers the Stenol-1822, powdered behenyl alcohol;
- the y-axis relates to the relative PEG level

The results show reduced dissolution of PEG with increasing levels of behenyl alcohol.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40mm" is intended to mean "about 40mm".

## Claims

1. A hair removal device comprising a solid lubricating composition, the solid lubricating composition comprising:
a. from 40% to 95% by weight of the solid lubricating composition of a water-soluble lubricating polymer which:
i. is solid at STP;
ii. has a molecular weight of less than or equal to 8,000,000, preferably from 100,000 to 3,000,000;
b. from 3 to 50%, preferably from 5 to 25%, by weight of the solid lubricating composition of one or more fatty alcohols, selected from C₁₂ - C₃₀ fatty alcohols.

2. The hair removal device of claim 1, wherein the fatty alcohol comprises cetyl, stearyl, or behenyl alcohol or mixtures thereof.

3. The hair removal device of claim 1 or 2, wherein the solid lubricating composition comprises less than 2%, preferably less than 1% and more preferably 0% of water-soluble surfactant, by weight of the solid lubricating composition.

4. The hair removal device of any preceding claim, wherein the water-soluble lubricating polymer comprises polyoxyalkylene glycol, polyalkane oxide, cellulosic polymer, polyvinyl pyrrolidone, polyacrylamide, polyvinyl imidazoline, polyhydroxyethylmethacrylate or mixtures thereof.

5. The hair removal device of any preceding claim, wherein the solid lubricating composition additionally comprising a hydrophobic binder.

6. The hair removal device of claim 5, wherein the hydrophobic binder comprises magnesium stearate, calcium stearate, zinc stearate, polycaprolactone, polyethylene, polypropylene, polystyrene, butadiene-styrene copolymer, polyacetal, acrylonitrilebutadiene-styrene copolymer, ethylene vinyl acetate copolymer, polypropylene/polystyrene blends or mixtures thereof.

7. The hair removal device of any preceding claim, wherein the solid lubricating composition comprises fragrance oil releasably encapsulated within a cyclic oligosaccharide.

8. The hair removal device of claim 7, wherein the cyclic oligosaccharide comprises α-cyclodextrin, β-cyclodextrin, methyl-α-cyclodextrin, methyl-α-cyclodextrin, hydroxypropyl-β-cyclodextrin, or mixtures thereof.

9. The hair removal device of any preceding claim, wherein the solid lubricating composition is cold-pressed and comprises less than or equal to 20%, preferably less than 15% of fatty alcohol by weight of the solid lubricating composition.

10. The hair removal device of any preceding claim, configured as a razor.

11. The hair removal device of any of claims 1 to 8, wherein the hair removal device comprises a light source to degrade or destroy the hair or hair root.

12. The hair removal device of claim 10, wherein the light source comprises a laser light source.

13. Use of a hair removal device according to any preceding claim to lubricate the skin and remove hair.
